# EUROPEAN PATENT APPLICATION

(11) **EP 1 103 607 A1**
(43) Date of publication of application: **30.05.2001**
(21) Application number: 99123363.6
(22) Date of filing: 23.11.1999
(51) Int. Cl.: C12N 15/12, A61K 38/17, A61K 38/19, A61K 48/00, C12N 5/10, G01N 33/50

(54) **Enhancement of tmTNF levels for the sensitisation of tumours to sTNF and/or cytostatic drugs**

(71) Applicant: Max-Planck-Gesellschaft zur Förderung der Wissenschaften e.V., Berlin (DE)
(72) Inventor: Claus, Matthias, 61231 Bad Nauheim (DE); Risau, Werner, Verstorben (DE); Sveinbjoernsson, Baldur, Tromsoe (NO); Grell, Matthias, 70771 Leinfelden/ (DE)
(74) Representative: VOSSIUS & PARTNER

(57) **Abstract**

Described are pharmaceutical compositions comprising a compound which is capable of effecting an increased endothelial level of tmTNF, preferably this increase of the endothelial level of tmTNF is restricted to tumors. Increase of endothelial level of tmTNF results in an increase of VEGF-induced hyperpermeability leading to sensitization of tumors for the application of sTNF, thus allowing the treatment of tumors, which can so far only be treated with extremely high doses of sTNF or which are resistant to such a treatment. In addition, pharmaceutical compositions are described additionally containing a cytostatic drug, since by increasing endothelial hyperpermeability, not only a drastic improvement of the delivery of sTNF to the tumor can be achieved, but in addition this also allows to more efficiently deliver cytostatic drugs to the tumor. Finally, kits for the diagnosis of tumors showing a reduced level of endothelial tmTNF are described.

## Description

The present invention relates to pharmaceutical compositions comprising a compound which is capable of effecting an increased endothelial level of transmembrane TNF (tmTNF), preferably this increase of the endothelial level of tmTNF is restricted to tumors. Increase of endothelial level of tmTNF results in an increase of vascular endothelial growth factor (VEGF)-induced hyperpermeability leading to sensitization of tumors for the application of soluble TNF (sTNF), thus allowing the treatment of tumors, which can so far only be treated with extremely high doses of sTNF or which are resistant to such a treatment. Compared to the exsection of the tumor this approach has various advantages, e.g. (a) inoperable tumors and disseminated metastases can be treated, (b) by avoiding exsection the risk of inducing the formation of metastases is drastically reduced, and (c) the induction of tumor necrosis by sTNF leads to a stimulation of an immune response directed against the tumor. Since by increasing the level of endothelial tmTNF hyperpermeability is generated, not only a drastic improvement of the delivery of sTNF to the tumor can be achieved, but in addition this also allows to more efficiently deliver cytostatic drugs to the tumor. Accordingly, the present invention also relates to pharmaceutical compositions additionally containing sTNF and/or a cytostatic drug. Finally, the present invention relates to kits for the diagnosis of tumors showing a reduced level of endothelial tmTNF.

VEGF is an essential inducer of angiogenesis observed during embryonic development and in many solid tumors (1). VEGF can also induce vascular permeability in vivo (2) and in vitro (3) which led to its initial discovery as vascular permeability factor (VPF). VEGF was also independently purified based on its ability to induce procoagulant activation of endothelial cells in vitro (4). This activation was suggested to participate in the sensitization of the tumor vasculature in certain experimental tumors, which is necessary for the induction of hemorrhagic necrosis by the tumor necrosis factor (TNF) (5-7). TNF is a pleiotropic cytokine, known to mediate in addition to its necrotizing activity towards tumors inflammatory, immunological and various pathophysiological reactions (8). It is produced predominantly by activated macrophages and lymphocytes and to a lesser extent also by other cells as a 26 kDa transmembrane protein (tmTNF), which is proteolytically processed (9) to the soluble 17 kDa form of TNF (sTNF). Whereas most biological effects exerted by sTNF are attributed to the 60 kDa TNF-receptor I (TNFRI), tmTNF is able to stimulate both TNF receptors including the 80 kDa TNFRII (10). It is known that the endothelial cell-mediated necrotizing activity of sTNF leads to a dramatic increase of the permeability and sometimes even hemorrhagic necrosis in tumors.
So far many tumors with metastasis (e.g. melanomas) are incurable. Although sTNF can in principal be used in order to treat tumors due its necrotizing activity described above, unfortunately, due to its high toxicity it can only applied to tumors being located in limbs. Moreover, most of the tumors (particularly human tumors) are resistant to a sTNF therapy.

Thus, the technical problem underlying the present invention is to provide means which allow to sensitize tumors for sTNF treatment.

The solution to the above technical problem is achieved by providing the embodiments characterized in the claims. It has been found that endogenous TNF is required for the induction of vascular permeability by VEGF not only in vitro but also in vivo. This activity is mediated by the transmembrane form of TNF (tmTNF) because an inhibitor of TNF-release from cells (TAPI) even increased the VEGF-mediated permeability in vivo. This specific effect of tmTNF on VEGF-induced permeability contributes to the vascular hyperpermeability of tumors and, thus, participates in the induction of hemorrhagic necrosis by sTNF. The in vitro studies demonstrate that VEGF-induced hyperpermeability (but not increased cell proliferation) is essentially dependent on tmTNF. Most interestingly, the in vivo studies showed that tmTNF is upregulated on the endothelium of the murine methylcholanthrene (meth A)-induced sarcoma versus normal dermal vasculature resulting in VEGF-induced hyperpermeability. This VEGF-induced hyperpermeability can be inhibited by treatment with anti-tmTNF antibodies in vivo and is absent in TNF knockout mice. Since the meth A-sarcoma can be efficiently treated by the application of sTNF it has to be concluded that the endothelial expression of tmTNF is responsible for the sensitization of the tumor for the induction of tumor necrosis by sTNF. Thus, tumors can be successfully treated by specifically increasing the concentration of tmTNF on the endothelium of the tumor (optionally in combination with the application of cytostatic drugs). This tmTNF-induced sensitization of tumors for the application of sTNF allows the treatment of tumors, which can so far only be treated with extremely high doses of sTNF or which are resistant to such a treatment. Compared with the exsection of the tumor this approach has various advantages, e.g. (a) inoperable tumors and disseminated metastases can be treated, (b) by avoiding exsection the risk of inducing the formation of metastases is drastically reduced, and (c) the induction of tumor necrosis by sTNF leads to a stimulation of an immune response directed against the tumor. In addition, the increased concentration of tmTNF in the VEGF producing tumor also results in an increased permeability for cytostatic drugs and, thus, allows to further improve the treatment of the tumor.

Thus, in one aspect, the present invention relates to a pharmaceutical composition comprising a compound which is capable of effecting an increased endothelial level of tmTNF, optionally in combination with a suitable pharmaceutical carrier. Preferably, the pharmaceutical compositions of the invention comprise a compound which is capable of specifically effecting an increased level of tmTNF on the endothelium of tumors. The person skilled in the art can select such compounds by well known methods and can evaluate whether said compounds have the desired activity, e.g. using the assay described in Examples 3 and 4, below. Examples of such compounds are the cDNA for TNF itself, small molecular compounds which increase TNF-expression on either transcriptional or post transcriptional levels and small molecular compounds which inhibit the TNF cleaving enzyme. Both the gene as well as the cDNA comprising the message for TNF have been cloned (for review see (11)) and can be used for both gene therapy (for example viral vectors carrying the TNF-cDNA) and the development of small molecular compounds which lead to transcriptional or post transcriptional activation of the TNF-gene (for this purpose 5'and 3' untranslated region would be attached to a reporter gene such as luciferase). Small molecular compounds which inhibit the conversion of tmTNF to soluble TNF are known (12) and comprise an alternative strategy to locally enhance the concentration of tmTNF. The cDNA can be targeted to the tumor by viral gene transfer or liposomes as a carrier. Small molecular compounds which enhance tmTNF expression can be applied by methods which are state of the art including the use of liposome formulations. Specificity for the tumor vasculature of these carriers can be achieved by attachment of antibodies/ligands specific for antigens/receptors which are upregulated on the endothelium of tumors such as the VEGF-receptor Flk-1.

Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the tumor and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of the tumor, general health and other drugs being administered concurrently.

As used herein the term _{"}a compound which is capable of effecting an increased endothelial level of tmTNF_{"} relates to a compound which increases the endothelial concentration of tmTNF in such a way that VEGF-induced hyperpermeability is increased and, as a consequence, uptake of compounds like sTNF and/or cytostatic drugs is improved. Preferably, this term includes compounds which can convert a tumor resistant to tmTNF therapy to a tumor which is susceptible of treatment with sTNF and/or a cytostatic drug.

In a preferred embodiment the pharmaceutical composition comprises a compound which is capable of increasing the expression of TNF. Such a compound comprises the DNA encoding TNF itself, either under a strong generally active or an endothelial specific promoter. Alternatively they comprise substances which can activate the TNF-promoter. Such substances can be cytokines such as II-1B or small chemical compounds. In this context transcription factors such as EGR-1, Ets-1, Jun or NF-kB had been described as inducers of the TNF-promoter (13-15) and could be used in assays to develop chemical compounds to activate the TNF-promoter, preferable in endothelial cells. Finally, upstream activators of transcription factor such as phorbol esters (14) or generators of free radicals such as menadione (2-methyl-1,4-naphtoquinone) (16) can be used either as drug or prodrugs targeted to the tumor vasculature by means of antibodies encoated liposomes or other techniques which are state of the art for vascular tumor therapy.

The present invention also provides pharmaceutical compositions comprising compounds suitable for gene therapy for the treatment of tumors by increasing the susceptibility of said tumors for sTNF and/or cytostatic drugs. Such therapy achieves its therapeutic effect by the introduction of a nucleic acid molecule encoding tmTNF into cells of patients with the tumor to be treated,. Thus, in a more preferred embodiment the pharmaceutical composition comprises a compound which is a nucleic acid molecule encoding (tm)TNF (17,18). By delivering this nucleic acid to the desired target, the intracellular expression of tmTNF and, thus, the endothelial level of tmTNF can be increased. Preferably, the nucleic acid encoding tmTNF is administered directly to the target site, e.g., by biolistic delivery, as a colloidal dispersion system or by catheter to a site in artery. The colloidal dispersion systems which can be used for delivery of the pharmaceutical composition of the invention include macromolecule complexes, nanocapsules, microspheres, beads and lipid-based systems including oil-in-water emulsions, (mixed) micelles, liposomes and lipoplexes. The preferred colloidal system is a liposome. The composition of the liposome is usually a combination of phospholipids and steroids, especially cholesterol. The skilled person is in a position to select such liposomes which are suitable for the delivery of the desired nucleic acid molecule. Organ-specific or cell-specific liposomes can be used in order to achieve delivery only to the desired tumor. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific tumors via specific cell-surface ligands. For example, certain antigens expressed specifically on tumor cells (tumor-associated antigens; TAAs) may be exploited for targeting liposomes to the malignant tumor. Liposomes with specificity for the tumor vasculature are currently in development. Alternatively, the liposomes can be targeted to the sites of tumors by attachment of anti-VEGF-receptor-antibodies to others antibodies directed against receptors or antigens which are upregulated in the tumor vasculature.

In a still more preferred embodiment, said nucleic acid molecule is integrated into a recombinant vector, preferably an expression vector. In order to achieve expression only in the target organ, i.e. the tumor to be treated, the nucleic acid molecule encoding tmTNF is operably linked to a tissue specific promoter and used for gene therapy. For example, the nucleic acid molecule encoding tmTNF can be ligated to the endothelial selective promoter tie-2. Alternatively, it could be ligated to the promoter of either one of the two VEGF-receptors, which are reportedly upregulated in the tumor vasculature.

Preferred recombinant vectors are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV), Rous sarcoma virus (RSV) and modified LENTI(HIV)-Viruses. Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotid encoding a sugar, a glycolipid, or a protein, preferably an antibody. In addition, endothelial specific promoters, which are in addition upregulated in the tumor vasculature such as the one of the VEGFR-2 (Flk-1,KDR), can be used in order to increase specificity. Those skilled in the art know additional methods for generating target specific vectors. Furthermore, adenovirus or adenovirus associated virus (AAV) are well described candidates for endothelial gene therapy. Care has to be taken, however, since adenoviruses tend to accumulate in lung and liver. Again, specificity to the tumor endothelium can be achieved by attachment of ligand or antibodies specific for proteins on the surface of the tumor endothelium such as the VEGFR-2 (Flk-1,KDR). Further suitable vectors and methods for in vitro- or in vivo-gene therapy are described in the literature and are known to the persons skilled in the art; see, e.g., WO 94/29469 or WO 97/00957. Preferably, the pharmaceutical composition further comprises an effective amount of sTNF in order to simultaneously effect the sensitization for treatment with sTNF (i.e. for enhancing the susceptability of a tumor for sTNF) and achieve effective treatment of the tumor with tolerable doses of sTNF. Tolerable doses are doses below 1 x 10⁶ E, preferably below 1 x 10⁵ E.

Due to the fact that by increasing the concentration of endothelial tmTNF hyperpermeability can be generated, not only a drastic improvement of the delivery of sTNF to the tumor can be achieved, but in addition this also allows to more efficiently deliver cytostatic drugs to the tumor. Thus, a further preferred embodiment of the present invention relates to a pharmaceutical composition further comprising a cytostatic drug. Preferred examples of such cytostatic drugs are melphalan, adriamycin, vincristine and related vinca alkaloids, cyclophosphamide, hydroxyurea, cis-platin and related substances.

Additionally, the present invention relates to a host cell comprising the above discussed vectors. The present invention also relates to the use of the above described compounds for the preparation of a pharmaceutical composition for the treatment of tumors, preferably tumors which are resistant to treatment with sTNF. Examples of tumors that can be treated by the pharmaceutical composition of the invention are mammary-, colon-, adeno- pancreas- hepatocyte- and lung-carcinoma, glioblastoma and sarcomas of muscle and connective tissue.

Finally, the present invention also relates to a kit for the diagnosis of tumors displaying low concentration of endothelial tmTNF comprising a TNF-receptor or a part thereof capable of binding TNF, an IgG-fusion protein, e.g. a soluble TNF-receptor (TNFR80) coupled to an immunglobulin molecule, or an anti-tmTNF-antibody or a fragment thereof, e.g., a f(ab) or f(ab)₂ fragment, which also specifically recognizes tmTNF. Such a kit can be used in order to determine whether a particular tumor shows low endothelial tmTNF concentration and, thus, is a good candidate for therapeutic intervention using the above described pharmaceutical compositions. In a preferred embodiment, said kit allows said diagnosis by ELISA and contains the antibody bound to a solid support, for example, a polystyrene microtiter dish or nitrocellulose paper, using techniques known in the art. Alternatively, said kits are based on a RIA and contain said antibody marked with a radioactive isotope. In a preferred embodiment of the kit of the invention the anti-tmTNF-antibody is labeled with enzymes, fluorescent compounds, luminescent compounds, ferromagnetic probes or radioactive compounds.

### Legends to the figures

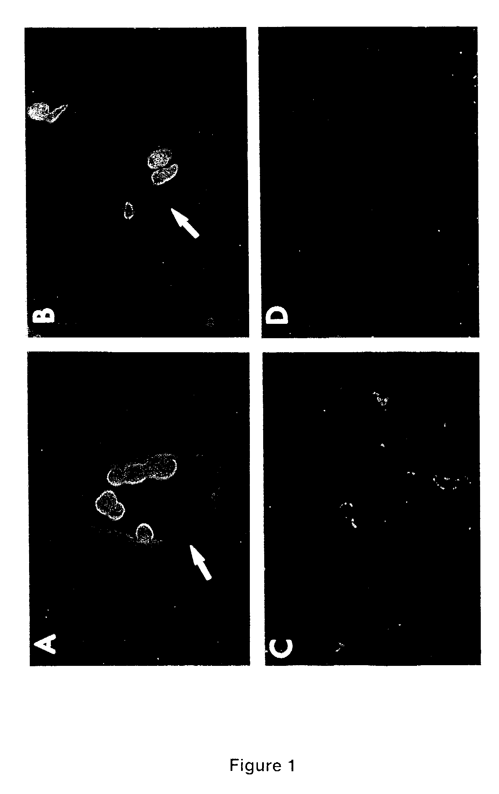

### Figure 1. Immunohistological analysis of endothelial TNF-expression.

**A:** TNF-staining on the endothelial lining (arrow) of the tumor-associated vasculature of transplanted Meth A-sarcomas. A strong autofluorescent reaction of some trapped red blood cells is also visible. **B:** Weak staining of blood vessels (arrow) for TNF can be observed in paraffin embedded dermal tissue distant from the tumor. **C, D:** Double staining of transmembrane TNF and CD31 as an endothelial cell marker in frozen sections of transplanted Meth A-sarcomas using a TNFR2-lgG fusion protein and a rat anti-mouse CD31-specific mAb. For further details see Example 1.

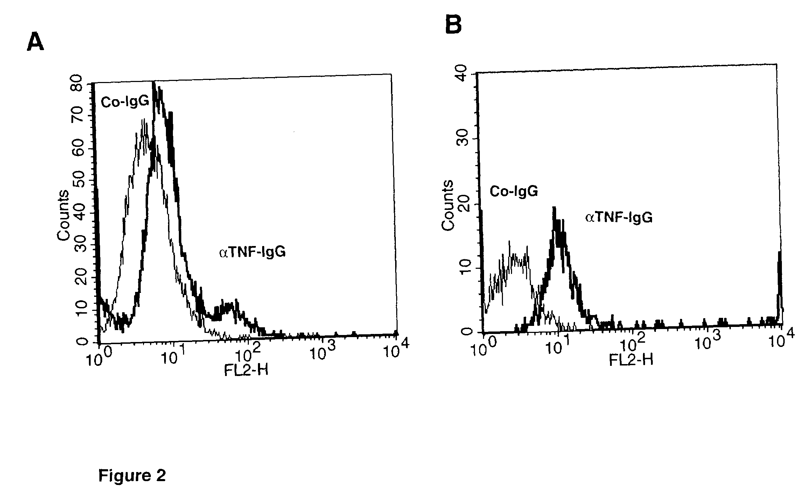

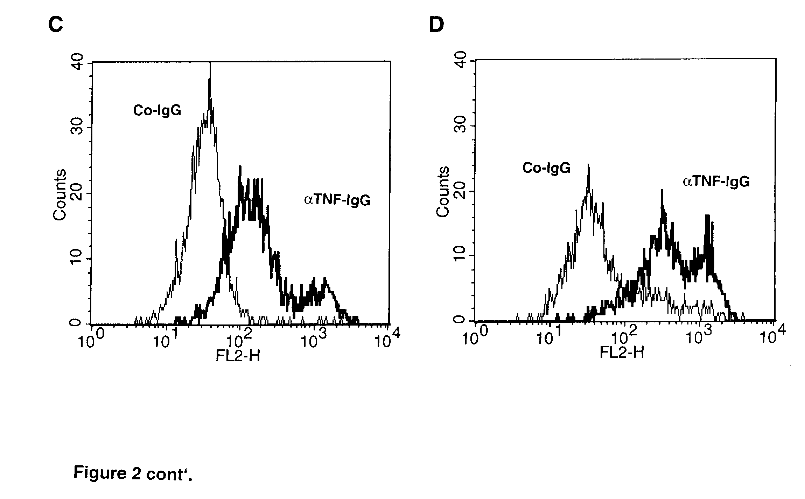

### Figure 2. Cytofluorimetric determination of endothelial tmTNF expression.

Primary HUVECs **(A)** of passage 3, primary human micro capillary endothelial cells **(B)** or immortalized HUVECs without **(C)** or with **(D)** acid wash. Flow cytometry analysis was performed on a _{"}FACStar_{"}. Isotype-matched control antibodies were used to determine the background staining. For further details see Example 2.

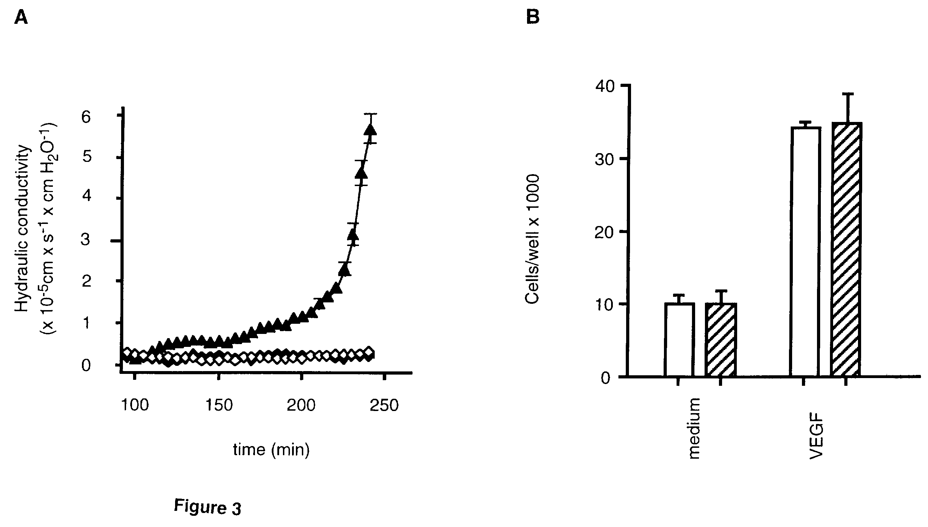

### Figure 3. TNF is permissive for VEGF-induced permeability of endothelial cell monolayers but not for VEGF-induced endothelial cell proliferation.

**A:** HUVEC were preincubated in the presence (closed diamonds) or absence (closed triangles) of the neutralizing TNF-specific Mab (357-101-4), which was described previously to neutralize human tmTNF (10), at a concentration of 10 mg/ml prior to treatment with VEGF (1 ng/ml). Values obtained from chambers not treated at all are indicated as open diamonds. **B:** HUVEC were cultured for 3 days with or without VEGF (10 ng/ml) in the presence (hatched bars) or absence (empty bars) of the neutralizing TNF-specific antibody (30 mg/ml). Data are presented as means (+/-SD) of three separate experiments.

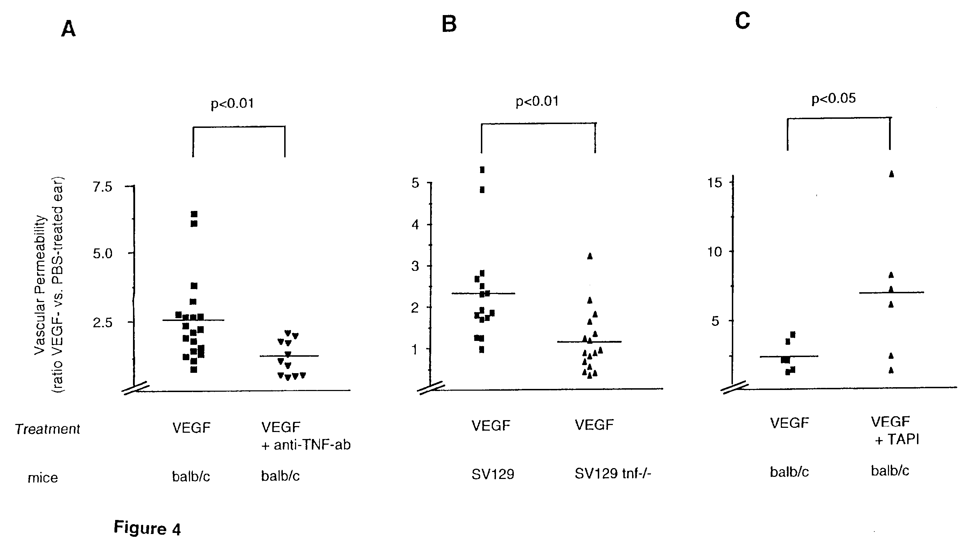

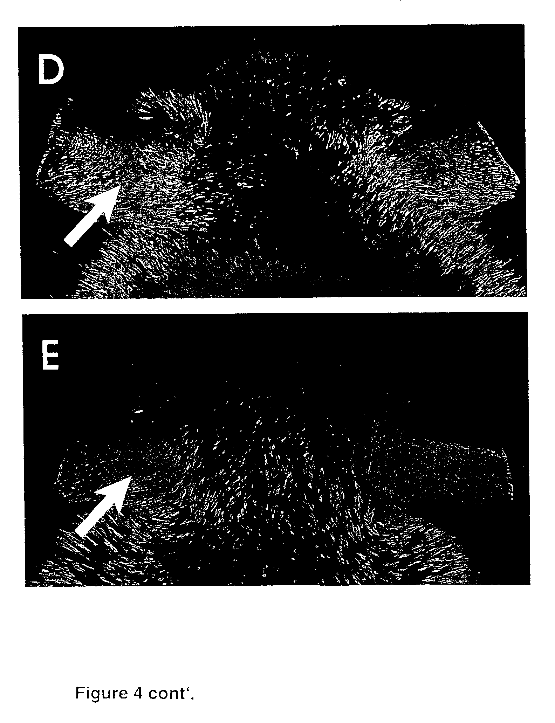

### Figure 4. TNF is permissive for VEGF-induced vascular permeability in vivo.

**A-C:** Shown are scatter diagrams with each symbol representing the ratio between the VEGF- and PBS-treated ear of one tested animal. Depicted are the mean (line) and the significance (p-values) as calculated from an unpaired t-test using a commercial program (InStat 2.01) of VEGF-induced vascular permeability in Balb/c mice with or without pretreatment with anti-TNF antibodies **(A)**, in TNF-deficient (tnf-/-) and the corresponding wild-type mice (SV129) **(B)**, or after pretreatment with TAPI or solvent **(C).** Please note the different y-axis scales. **D,E:** VEGF-induced vascular permeability was determined in wild-type (**D)** and TNF-deficient mice **(E)** after i.v. injection of Evans blue dye and subsequent dermal injection of VEGF into the left ear (arrows) and of PBS into the right ear.

The following examples illustrate the invention.

### Example 1: Immunohistological analysis of the endogenous TNF expression

Based on the reasoning that endothelial cells produce TNF upon stimulation and that tumor vasculature is in a preactivated and procoagulant status sections from transplanted meth A-sarcomas were analysed for endogenous TNF expression. It was found that TNF is strongly expressed in the endothelial lining of the tumor vasculature by using anti-TNF antibodies (Fig.1A; arrow) or a TNFRI-lgG fusion protein (Fig.1C). Specific staining of the endothelium was demonstrated by double staining with an anti-PECAM antibody (Fig. 1 D). Vasculature of normal dermis distal from the tumor showed only faint or no staining (Fig.1B); arrow), whereas vessels from other organs tested (lung and liver) did not stain with the same anti-TNF antibodies (data not shown). The staining of the tumor vessels is on the endothelium because double staining with an TNFRI-lgG- fusion protein (5 mg/ml) (Fig.1C) and anti-CD31 mAb (Fig.1 D) which recognizes the endothelial marker protein PECAM-1 revealed an identical staining pattern on meth A sarcoma sections.

Experimental details: Sections from transplanted meth A-sarcomas were analysed for endogenous TNF expression using a TNFRI-lgG fusion protein. Meth A-sarcoma were perfusion-fixed with 4% paraformaldehyde and embedded in paraffin (19). Sections of tumors and other tissues were stained with antibody using indirect immunohistochemistry. Polyclonal anti-mouse TNF (Genzyme Virotech, Russelsheim, Germany) antibodies (1:100 diluted serum) were visualized with Cy5-conjugated goat anti-rabbit antibodies (diluted 1:1000; Dianova, Hamburg, Germany). Cryostat sections from the same type of tumor were fixed with cold acetone and stained with TNFR2-hulgG fusion protein (20) (10 mg/ml) and visualized by using rabbit anti-human IgG antibody (diluted 1:100; Cappel, Durham, NC), biotinylated goat anti-rabbit IgG antibody (diluted 1:250; Boehringer, Mannheim, Germany) and FITC-labeled streptavidin (diluted 1:1000; Zymed, San Francisco, CA). Rat anti-mouse CD31 (21) (1 mg/ml) was visualized with TRITC-conjugated goat anti-rat IgG (diluted 1:25; Zymed).

### Example 2: Cytofluorometric analysis of primary endothelial cell-types for the presence of tmTNF

In a first attempt to understand the role of TNF within the vessels of meth A-sarcomas, it was looked for an appropriate model in vitro. Thus, various primary endothelial cell types were tested for the presence of tmTNF by cytofluorometric analysis with the monoclonal anti-TNF antibody. This antibody has been shown to be selective for tmTNF versus sTNF at low antibody concentrations. The analysis of human umbilical vein endothelial cells (HUVECs) by FACS with the ANTI-TNF antibody revealed a heterogeneous staining pattern (Fig.2A): most of the cells showed a weak reaction with the anti-TNF-antibody (left peak) with approximately 10% of cells staining strongly (the second smaller peak). In addition to HUVECs, other primary endothelial cells, i.e., human umbilical arterial endothelial cells and dermal human microvascular endothelial cells (Fig.2B) showed surface expression of tmTNF. The ANTI-TNF-antibody staining of endothelial cells did not appear to be caused by receptor bound soluble TNF since acid washing (pH 2.1), able to significantly reduce the levels of receptor bound soluble TNF, was ineffective (Fig.2C+D).

Experimental details: Primary HUVECs of passage 3 (Fig.2A), human micro capillary endothelial cells (Cascade Biologics, Portland, OR)(Fig.2B) or immortalized HUVECs (ATCC#CRL1730, Washington, DC) without (Fig.2C) or with (Fig.2D) acid wash (pH 2.1) were used. For the acid wash treatment cells were incubated for 5 min. on ice with isotonic solution with the pH adjusted to 2.1 by addition of HCI to glycine (100 mM)buffer. Cells treated or untreated were detached with 5 mM EDTA in HBSS/25 mM HEPES and incubated with primary unconjugated anti-TNF (bold line) for 30 min. at 4°C and 5 mg/ml as a final concentration, washed twice with FACS-buffer (PBS supplemented with 1% BSA and 0,1% NaN₃) and then incubated with PHE-conjugated goat anit-mouse IgG for 30 min. at 4°C. Flow cytometry analysis was performed on a _{"}FACStar_{"} (Becton Dickinson Immunocytochemistry Systems, Mountain View, CA, USA). Isotype-matched control antibodies (mlgG, normal line) were used to determine background staining.

### Example 3: Effect of neutralizing anti-tmTNF-antibodies on VEGF-induced endothelial monolayer permeability and proliferation

Both VEGF and soluble TNF have been described to cause both vascular leakage (2, 22) and angiogenesis (23,24). In a previous study it was shown that TNF is expressed on the tumor vasculature. Thus, it was hypothesized that TNF cooperates with soluble VEGF to cause hyperpermeability and angiogenesis. To test this hypothesis the involvement of TNF in VEGF-induced endothelial permeability and proliferation in vitro was tested. VEGF-dependent increase of endothelial monolayer permeability was determined in vitro by measuring the hydraulic conductivity of endothelial monolayers grown on polycarbonate filter membrans as described recently (3,25). VEGF-induced endothelial hyperpermeability was completely inhibited by pre-incubation of cells wich express cellular TNF with a TNF-specific neutralizing monoclonal antibody (Fig.2A and inset). No change in hydraulic conductivity was noted in control cell monolayers over a period of 4 hours. Subsequent addition of a-toxin, a well established permeability increasing agent (25), to these cells resulted in a rapid increase in the water filtration rate, indicating the well preserved reactivitiy of the endothelial cell monolayers. This modulation of VEGF is most likely caused by the transmembrane form of TNF (tmTNF) because sTNF could not be detected in the supernatant of endothelial cells by the use of either ELISA (R&D-systems) or a cytotoxicity assay using L929 cells. In contrast to the results obtained by measuring endothelial permeability, the anti-TNF antibody did not effect endothelial proliferation, either in the absence or in the presence of VEGF, although VEGF was clearly able to stimulate an increase in endothelial cell numbers (Fig.3B). This clearly demonstrates that tmTNF is essentially involved in VEGF mediated endothelial permeability induction but not in mitogenic effects of VEGF.

Experimental details: To test monolayer permeability confluent HUVECs (P3), isolated and cultivated as described previously (3), were grown on polycarbonate filters, mounted in a modified chemotaxis chamber and changes in hydraulic conductivity by VEGF determined under a hydrostatic pressure of 10 cm H₂O as described recently (3,14). Briefly, ³H₂O was applied together with rhVEGF³ (1 ng/ml) to the upper chamber and the transflux of tracer in the lower chamber was continuously measured with a Ramona LS-5 radioactivity monitor (Raytest, Heidelberg, Germany). For experiments with neutralizing antibodies, cells were preincubated for 20 min at 37°C prior to the addition of reagents. For determination of endothelial proliferation, HUVEC were seeded in 24 well-plates (6000 cells/well) and subsequently cultivated for 3 days with or without VEGF (10 ng/ml) in the presence or absence of antibodies. Cells were then detached by treatment with trypsin and cell numbers were counted in a Casy-cell counter (Schärfe-System, Reutlingen, Germany).

### Example 4: Analysis of the effect of tmTNF on VEGF induced vascular permeability in vivo

In order to assess whether the essential role of TNF for the activity of VEGF in endothelial hyperpermeability is also relevant in vivo, we used the model of a modified Miles assay of vascular permeability. In this assay i.v. injection of FITC-conjugated albumin in mice was followed by an intradermal injection of VEGF and extravasation of fluorochrom was quantitatively measured. Similar to the situation in vitro, an antiserum capable of neutralizing tmTNF completely blocked the ability of VEGF to induce hyperpermeability (Fig.4A). Furthermore, TNF gene-deficient mice (26) failed to respond to the permeability increasing activity of VEGF, whereas in wild type mice of the same strain VEGF was active (Fig.4B). This modulation of VEGF dependent increase in vascular permeability is most likely due to membran-TNF and not soluble TNF because pretreatment with a metalloproteinase inhibitor (TAPI), shown to selectively block the generation of soluble TNF in vivo, did not alter the response of VEGF.

Experimental details: Mice were injected i.v. with 2.5 mg FITC-labeled BSA (Sigma, Munich, Germany) in 100 ml PBS. After 3 min, mice were anesthesized and VEGF (0.2 mg in 20 ml PBS) or solvent (PBS) were injected into the left and right ear, respectively. Six minutes later, the animals were sacrificed and the extravasated fluorescent dye was extracted from the ears and measured using a fluorescence photometer. In some experiments mice were pretreated i.p. with polyclonal anti-TNF serum (100 ml; Genzyme) or saline for 10 min or, alternatively, for 20 min with the TNF-selective metalloproteinase inhibitor TAPI (500 mg/animal) or solvent (DMSO). In a limited number of experiments Evans blue dye (1.2 mg in 100 ml PBS) was used instead of FITC-labeled BSA. After treatment, extravasation of the dye was documented by photography.

### REFERENCES

1. W. Risau, *Nature* **386**, 671 (1997).
2. D. Senger, S. Galli, A. Dvorak, C. Peruzzi, V. Harvey, et al, *Science* **219**, 983 (1983).
3. S. Hippenstiel, M. Krüll, A. Ikemann, W. Risau, M. Clauss, et al, *Am. J. Physiol.* **274**, L678 (1998).
4. M. Clauss, M. Gerlach, H. Gerlach, J. Brett, F. Wang, et al, *J. Exp. Med.* **172**, 1535(1990).
5. M. Clauss, J. Ryan, D. Stern, in *Tumor Necrosis Factors,* Bruce Beutler, Ed. (Raven Press, New York, 1992), p. 49.
6. K. Shimomura, S. Manda, K. Mukomoto, Nakano,K., J. Mori, *Int. J. Cancer* **41**, 243 (1988).
7. E. A. Havell, W. Fiers, R. J. North, *J. Exp. Med.* **167**, 1067 (1988).
8. W. Fiers, *FEBS Lett.* **285**, 199 (1991).
9. R. A. Black, C. T. Rauch, C. J. Koziosky, J. J. Peschon, J. L. Slack, et al, *Nature* **385**, 729 (1997).
10. M. Grell, E. Douni, H. Wajant, M. Löhden, M. Clauss, et al, *Cell* **83,** 793 (1995).
11. Roth J., Gehr, G., Loetscher H., Lesslauer W. *Immunologic Research* **11(2)**, 81-90 (1992)
12. Mohler K.M., Sleath P.R., Fitzner J.N., Cerretti D.P., Alderson M. et al. *Nature* **370**,218(1994)
13. Kramer B., Wiegmann K. & Kronke M. *Journal of Biological Chemistry* **270,** 6577 (1995)
14. Kramer B., Meichle A., Hensel G., Charnay P. & Kronke M. *Biochimica et Biophysica Acta* **1219**, 413 (1994).
15. Udalova I.A., Knight J.C., Vidal V., Nedospasov S.A. & Kwiatkowski D. *Journal of Biological Chemistry* **273**, 21178 (1998).
16. Lewartowski B., Zdanowski K. & Wolska B.M. *Journal of Physiology & Pharmacology* **42**, 221 (1991 ).
17. Shirai T., Yamaguchi H., Ito H., Todd C.W. & Wallace R.B. *Nature* **313,** 803(1985).
18. Pennica D., Hayflick J.S., Bringman T.S., Palladino M.A. & Goeddel D.V. *Proc. Nat. Acad. Sc. USA* **82,** 6060 (1985)
19. R. Seljelid, *Scand. J. Immunol.* **29,** 181 (1989).
20. D. Moosmayer, H. Wajant, E. Gerlach, M. Schmidt, B. Brocks, et al, J. *Interferon&Cytokine. Res.* **16,** 471 (1996).
21. Vecchi, A. et al. *Eur. J . Cell Biol.* **63,** 247 (1994).
22. Y. Abe, S. Sekiya, T. Yamasita, F. Sendo, *J. Immunol.* **145**, 2902 (1990).
23. B. Millauer, L. Shawver, K. H. Plate, W. Risau, A. Ullrich, *Nature* **367,** 576 (1994).
24. M. Frater-Schröder, W. Risau, R. Hallmann, P. Gautschi, P. Böhlen, *Proc. Natl. Acad. Sci. USA* **84,** 5277 (1987).
25. N. Suttorp, U. Weber, T. Welsch, C. Schudt, *J. Clin. Invest.* **91,** 1421 (1993).
26. M. W. Marino, A. Dunn, D. Grail, M. Inglese, Y. Noguchi, et al., *Proc. Natl. Acad. Sci. USA* **94,** 8093 (1997).

## Claims

1. A pharmaceutical compositon comprising a compound which is capable of effecting an increased endothelial level of tmTNF, optionally in combination with a suitable pharnmaceutical carrier.

2. The pharmaceutical composition of claim 1, wherein said compound is a compound which is capable of increasing the expression of tmTNF.

3. The pharmaceutical composition of claim 2, wherein said compound is a nucleic acid sequence encoding tmTNF.

4. The pharmaceutical composition of claim 2, wherein said compound is a vector comprising the nucleic acid sequence of claim 3.

5. The pharmaceutical composition of claim 4, wherein said vector is an expression vector.

6. The pharmaceutical composition of claim 5, wherein said vector is a retro- or adenoviral vector.

7. The pharmaceutical composition of any one of claims 1 to 6 further comprising an effective amount of sTNF and/or a cytostatic drug.

8. The pharmaceutical composition of claim 7, wherein the cytostatic drug is melphalan, adriamycin, vincristine or a related vinca alkaloid, cyclophosphamide, hydroxyurea or cis-platin.

9. A host cell comprising the vector as defined in any one of claims 4 to 6.

10. Use of the compound(s) as defined in any one of claims 1 to 6 for the preparation of a pharmaceutical composition for enhancing the susceptability of a tumor for systemic treatment with tolerable doses of sTNF and/or a cytostatic drug.

11. Use of the compound(s) as defined in claim 7 or 8 for the preparation of a pharmaceutical composition for the treatment of a tumor.

12. Use according to claim 10 or 11, wherein the tumor is a tumor resistant to systemic treatment with tolerable doses of sTNF.

13. Use according to claim 12, wherein the tumor is a mammary-, colon-, adeno-pancreas- hepatocyte-, lung-carcinoma, glioblastoma or a sarcoma of muscle and connective tissue.

14. A kit for the diagnosis of tumors displaying low levels of endothelial tmTNF comprising a TNF-receptor or a part thereof capable of binding TNF, IgG-fusion protein or an anti-tmTNF-antibody or a fragment thereof.

15. The kit of claim 14, wherein the TNF-receptor or a part thereof capable of binding TNF, IgG-fusion protein or anti-tmTNF-antibody is labelled with enzymes, fluorescent compounds, luminescent compounds, ferromagnetic probes or radioactive compounds.
